# EUROPEAN PATENT SPECIFICATION

(11) **EP 1 501 554 B1**
(45) Date of publication and mention of the grant of the patent: **13.09.2017**
(21) Application number: 03722601.6
(22) Date of filing: 02.05.2003
(51) Int. Cl.: A61K 51/08, A61P 35/00

(54) **BENZOTHIENYL ANALOGUE OF SOMATOSTATINE, SELECTIVE FOR CERTAIN SOMATOSTATIN RECEPTORS**
BENZOTHIENYL-ANALOGE VON SOMATOSTATIN, SELEKTIV FÜR BESTIMMTE SOMATOSTATINREZEPTOREN
DERIVES BENZOTHIENYLE DE LA SOMATOSTATINE, SELECTIFS POUR CERTAINS RECEPTEURS DE LA SOMATOSTATINE

(30) Priority: 03.05.2002 EP 02076757
(43) Date of publication of application: 02.02.2005
(73) Proprietor: Advanced Accelerator Applications USA, Inc., Wilmington, DE 19808 (US)
(72) Inventor: MAECKE, Helmut, Robert, 79541 Loerrach (DE); REUBI, Jean, Claude, CH-3084 Wabern (CH); DE JONG, Marion, NL-3137 TN Vlaardingen (NL); KRENNING, Eric, Paul, NL-3062 JC Rotterdam (NL); GINJ, Mihaela, 4058 Basel (CH)
(74) Representative: Hooiveld, Arjen Jan Winfried
(86) International application number: PCT/EP2003/004847
(87) International publication number: WO 2003/092744

(56) References cited:
- WO-A-01/81426
- WO-A-97/01579
- WO-A-98/41540
- WO-A-02/100885
- US-A- 5 750 499
- US-B1- 6 262 229
- M. GINJ ET AL.: "DOTA-NOC, A HIGH AFFINITY LIGAND FOR SOMATOSTATIN RECEPTOR SUBTYPES 2, 3 AND 5" FIFTH INTERNATIONAL ELECTRONIC CONFERENCE ON SYNTHETIC ORGANIC CHEMISTRY (ECSOC-5), 1-30 SEPTEMBER 2001, [Online] 2 September 2001 (2001-09-02), XP002234587 Internet Retrieved from the Internet: <URL:http://www.mdpi.net/ecsoc-5/c0035/c00 35-Dateien/frame.htm> [retrieved on 2003-01-28] & O. KAPPE ET AL. (EDS.): "Proceedings of ECSOC-5" [CD-ROM] 2001, MOLECULAR DIVERSITY PRESERVATION INTERNATIONAL (MDPI) , BASEL, CH ISBN: 3-906980-06-5
- M. GINJ ET AL.: "DOTA-NOC, A HIGH AFFINITY LIGAND FOR SOMATOSTATIN RECEPTOR SUBTYPES 2, 3 AND 5" EUROPEAN JOURNAL OF NUCLEAR MEDICINE, vol. 28, no. 8, 2001, page 966, abstract no. OS_24, XP002234588 ISSN: 0340-6997 cited in the application
- ROSSOWSKI WOJCIECH J ET AL: "Potent inhibitory effects of a type four receptor-selective somatostatin analog on rat insulin release." BIOCHEMICAL AND BIOPHYSICAL RESEARCH COMMUNICATIONS, vol. 197, no. 2, 15 December 1993 (1993-12-15), pages 366-371, XP001133781 ISSN: 0006-291X
- REUBI J C ET AL: "AFFINITY PROFILES FOR HUMAN SOMATOSTATIN RECEPTOR SUBTYPES SST1- SST5 OF SOMATOSTATIN RADIOTRACERS SELECTED FOR SCINTIGRAPHIC AND RADIOTHERAPEUTIC USE" EUROPEAN JOURNAL OF NUCLEAR MEDICINE, BERLIN, DE, vol. 27, no. 3, March 2000 (2000-03), pages 273-282, XP001042272 ISSN: 0340-6997
- FAGAN SHAWN P ET AL: "Insulin secretion is inhibited by subtype five somatostatin receptor in the mouse." SURGERY (ST LOUIS), vol. 124, no. 2, August 1998 (1998-08), pages 254-259, XP008013242 ISSN: 0039-6060
- GINJ M ET AL: "Synthesis and biological activity of a new and highly potent ligand for somatostatin receptors 2, 3 and 5." JOURNAL OF PEPTIDE SCIENCE, vol. 8, no. Supplement, August 2002 (2002-08), page S68, abstract no. L46, XP008013180 & 27th European Peptide Symposium;Sorrento, Italy; August 31-September 06, 2002, 2002 ISSN: 1075-2617
- SCHMITT J S ET AL: "DOTA-NOC, a high affinity ligand of the somatostatin receptor subtypes 2, 3 and 5 for radiotherapy." JOURNAL OF LABELLED COMPOUNDS AND RADIOPHARMACEUTICALS, vol. 44, no. Supplement 1, May 2001 (2001-05), pages S697-S699, XP008013182 Fourteenth International Symposium on Radiopharmaceutical Chemistry;Interlaken, Switzerland; June 10-15, 2001 ISSN: 0362-4803
- BRUNS CHRISTIAN ET AL: "Binding properties of somatostatin receptor subtypes." METABOLISM CLINICAL AND EXPERIMENTAL, vol. 45, no. 8 SUPPL. 1, 1996, pages 17-20, XP008013183 ISSN: 0026-0495
- LEWIS, IAN ET AL: "Synthesis of somatostatin analogs incorporating nucleo amino acids" TETRAHEDRON, vol. 50, no. 25, 1994, pages 7485-7494, XP001109510
- PINSKI JACEK ET AL: "Somatostatin analog RC-160 and bombesin/gastrin-releasing peptide antagonists RC-3095 inhibit the growth of androgen-independent DU-145 human prostate cancer line in nude mice." CANCER LETTERS, vol. 71, no. 1-3, 1993, pages 189-196, XP008013243 ISSN: 0304-3835
- PLISKA VLADIMIR ET AL: "Side chain lipophilicity of noncoded .alpha.-amino acids: .pi.-values", PRINCIPLES OF MEDICINAL CHEMISTRY, vol. 4, 1 January 1996 (1996-01-01), pages 375-386, XP008080327,
- DE JONG M ET AL: "Comparison of <111>In-labeled somatostatin analogues for tumor scintigraphy and radionuclide therapy", CANCER RESEARCH, vol. 58, no. 3, 1 February 1998 (1998-02-01), pages 437-441, ISSN: 0008-5472

## Description

The invention relates to a peptide compound having an improved binding affinity to somatostatin receptors, comprising a peptide and a chelating group covalently linked to a free amino group of said peptide.

Such peptide compounds and their radiolabelled derivatives can be used for therapy of somatostatin - receptor positive tumors. Detectably labelled somatostatin - peptide compounds are also useful for in vivo imaging. See in this respect the patent publications of Albert et al. (US 5,753,627; US 5,776,894), of Krenning et al. (US 6,123,916), of De Jong et al. (WO 00/18440), of Srinivasan et al. (US 5,804,157; 5,830,431), and recent international patent application PCT/EP01/04764. Albert et al. disclose complexed somatostatin peptides for in vivo imaging of somatostatin receptor - positive tumors, which peptides are derived from somatostatin analogues, carrying an optionally substituted phenylalanine residue or a beta- or 2-naphthylalanine residue in its 3-position.
Selective internal tumor therapy with radiolabelled peptides has become very important in nuclear medicine in the past years. Especially somatostatin derivatives have been successfully applied in the clinic for tumor diagnosis and therapy, showing that the principle of receptor targeting is working in practice. For more than four years already much experience has been gained in clinical trials with the use of ⁹⁰Y - labelled DOTA-[Tyr³]-octreotide (DOTA-TOC) for tumor therapy (M. de Jong: Eur. J. Nucl. Med. 26, 1999, 693-698).

Yet DOTA-TOC only shows high affinity to the somatostatin receptor subtype 2 (sst 2), whereas the affinity to other somatostatin subtypes, in particular sst 3 and sst 5, which are found also in a variety of tumors, is too low to contribute essentially to tumor targeting. For example, most thyroid tumors express these last-mentioned somatostatin receptor subtypes, but carry sst 2 only on a low level (E.B. Forssell-Aronsson et al.: J. Nucl. Med. 41, 2000, 636-642).

Recently, additional results of labelled octreotide analogues have been disclosed: J. Labelled Cpd. Radiopharm. 44, Suppl. 1 (2001), 5697-5699 (see also: Eur. J. Nucl. Med. 28/8, OS 24 (2001), 966). In this paper it is suggested, that modification of the 3-position of octreotide, especially with hydrophobic aromatic amino acids, could improve the sst 3 and sst 5 receptor affinities. It has indeed been found, according to this publication, that DOTA-[1-Nal³]-octreotide (DOTA-NOC), i.e. an octreotide analogue having in its 3-position 1-naphyhylalanine substituted for phenylalanine, has a significant affinity to sst 3 and sst 5 somatostatin receptors, attended by a superior sst 2 receptor affinity.
Still there remains a need for peptide compounds having a further improved binding affinity to a plurality of somatostatin receptors.

Other publications that disclose somatostatin derivatives which are based on octreotide compounds wherein the 3- position of Phe (1-phenylalanine) is replaced by another amino acid , are given in the following publications:
A replacement of Phe (1-phenylalanine) at the 3-position by 1-Nal or 2-Nal (naphthylalanine) is disclosed in: "DOTA-NOC, A HIGH AFFINITY LIGAND FOR SOMATOSTATIN RECEPTOR SUBTYPES 2, 3 AND 5", by Ginj M., et al, in: INTERNATIONAL ELECTRONIC CONFERENCE ON SYNTHETIC ORGANIC CHEMISTRY, 1-30 September 2001, Page(s):1 - 14.
A replacement of Phe (1-phenylalanine) at the 3-position by Trp (tryptophane) is disclosed in WO9701579 and in WO0181426.
A replacement of Phe (1-phenylalanine) at the 3-position by Tyr (tyrosine) is disclosed in: "AFFINITY PROFILES FOR HUMAN SOMATOSTATIN RECEPTOR SUBTYPES SST1-SST5 OF SOMATOSTATIN RADIOTRACERS SELECTED FOR SCINTIGRAPHIC AND RADIOTHERAPEUTIC USE", by Reubi J C, et al, in: EUROPEAN JOURNAL OF NUCLEAR MEDICINE, Vol:27,Nr:3, Page(s):273 - 282, March 2000.

It is therefore the objective of the present invention to provide a peptide compound which has an improved binding affinity to a plurality of somatostatin receptor subtypes, compared with the above known somatostatin peptides. On account of its multispecificity, such a peptide compound , in particular after labelling with a suitable radionuclide, could be therapeutically used to more effectively treat a broad variety of tumors. In addition, after labelling with a suitable detectable element, such a peptide compound should have an improved suitability for in vivo detecting and localizing tissues, in particular tumors and metastases thereof, carrying somatostatin receptor types in varying levels.

This objective can be achieved, according to the present invention, by a peptide compound as defined in the opening paragraph, wherein said peptide is or comprises a somatostatin analogue carrying a 3-benzothienylalanine residue in its 3-position, wherein the peptide has the general formula

H- (D)A¹-*cyclo*[Cys²-A³-A⁴-A⁵-A⁶-Cys⁷]-A⁸ (I)

wherein:
A¹ is optionally halogenated Tyr, or optionally halogenated or methylated (D)Phe or Nal,
A⁴ is (D)-Trp, optionally N-methylated in its side-chain,
A⁵ is Lys, optionally N-methylated in its side-chain,
A⁶ is Thr, Val, Ser, Phe or He, and
A⁸ is Thr, Trp or Nal, wherein the terminal carboxy group may be modified to an alcohol or an , optionally C1-C3 alkylated, amide group,
and characterized in that A³ is 3-benzothienylalanyl.

It has been found, that the new peptide compounds of the invention have an unexpectedly high affinity to a plurality of somatostatin receptor subtypes. These favourable results are against expectation, because the hydrophobic character of the peptide is not improved by substituting phenylalanine or 1-naphthylalanine by 3-benzothienylalanine. This favourable binding affinity makes the new peptide compounds promising candidates both for diagnosis, after labelling, and for tumor therapy. From biodistribution studies it can be concluded that the labelled new peptide compounds of the invention show a favourable target to non-target distribution in vivo.

Document D1, M Ginj et al.: "Dota-Noc, A high affinity ligand for somatostatin receptor subtypes 2, 3 and 5", Fifth International Electronic Conference on Synthetic Organic Chemistry, September 2001, discloses a corresponding peptide with 1-naphthylalamine in position 3.

Suitable examples of the above new peptides of formula I are:
(1) H-(D)Phe-cyclo[Cys-3-benzothienylalanyl-(D)Trp-Lys-Thr-Cys]-Thr-ol
(2) H-(D)Nal-*cyclo*[Cys-3-benzothienylalanyl-(D)Trp-Lys-Val-Cys]-Thr-NH₂
(3) H-(D)Phe-cyclo[Cys-3-benzothienylalanyl-(D)Trp-Lys-Thr-Cys]-Thr-OH

The above peptide 3 has the following structural formula:

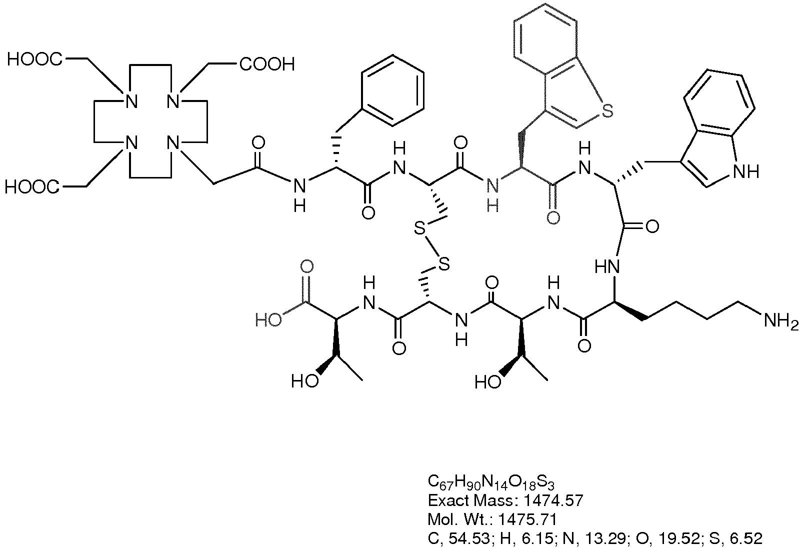

The above examples are covered by the general formula II, encompassing preferred peptides according to the invention.

H-(D)Á¹-*cyclo*[Cys²-A³-(D)-Trp⁴-Lys⁵-Á⁶-Cys⁷]-Á⁸ (II)

wherein:
Á¹ is optionally halogenated Tyr, or Phe or Nal,
A³ is 3-benzothienylalanyl
Á⁶ is Thr or Val, and
Á⁸ is Thr-ol, Thr-OH or Thr-NH₂.

The peptide compound according to the invention comprises a chelating group covalently linked to the N-terminal free amino group of the peptide. Various well-known chelating groups can be used, for example, those selected from:
(i) N₂S₂-, N₃S- and N₄-tetradentate ring structure containing groups, (ii) isocyanate, carbonyl, formyl, diazonium, isothiocyanate and alkoxycarbimidoyl containing groups, (iii) groups derived from N-containing di- and poly acetic acids and their derivatives, and from (iv) 2-iminothiolane and 2-iminothiacyclohexane containing groups.

The chelating groups sub (iii) above are preferred, encompassing chelating groups derived from ethylenediamine tetraacetic acid (EDTA), diethylenetriamine pentaacetic acid (DTPA), ethyleneglycol-OO'-bis(2-aminoethyl)-N,N,N',N'-tetraacetic acid (EGTA), N,N'-bis(hydroxybenzyl)ethylenediamine-N,N'-diacetic acid (HBED), triethylenetetramine hexaacetic acid (TTHA), substituted EDTA or DTPA, 1,4,7,10-tetraazacyclododecane-N,N',N",N'''-tetraacetic acid (DOTA), 1,4,7-triazacyclonane-1,4,7-triacetic acid (NOTA) or 1,4,8,11-tetraazacyclotetradecane-N,N',N",N"'-tetraacetic acid (TETA).

The method of linking the chelating group to a free amino group of the peptide for obtaining the peptide compound of the invention is generally known in the art. The synthesis of peptide compounds having the above chelating group sub (iv) is described in WO 89/07456. Peptide compounds wherein the peptide comprises a somatostatin analogue, more in particular wherein the peptide has the above general formula I, wherein n is 1 and A⁰ is a trifunctional amino acid residue to which an additional peptide part, comprising 3 to 12 amino acid residues which form at least once the Arg-Gly-Asp sequence, is covalently linked, can be prepared according to the above PCT/EP01/04764.

Generally, for the purpose in view, the peptide compound of the invention is labelled with a detectable element selected from gamma- or positron-emitting radionuclides, Auger-electron-emitting isotopes and paramagnetic ions of nonradioactive elements, or with a therapeutic radionuclide.

Suitable detectable elements for imaging purposes are gamma- or positron-emitting radionuclides, selected from the group consisting of ^{99m}Tc, ²⁰³Pb, ⁶⁶Ga, ⁶⁷Ga, ⁶⁸Ga, ⁷²As. ¹¹¹In, ^{113m}In, ⁹⁷Ru, ⁶²Cu, ⁶⁴Cu, ⁵²Fe, ⁵¹Cr, ²⁴Na, ¹⁵⁷Gd, ^{52m}Mn, ¹⁶²Dy, ¹²³I, ¹³¹I, ⁷⁵Br and ⁷⁶Br, or paramagnetic ions of elements, selected from the group consisting of nonradioactive Gd, Fe, Mn and Cr.

In addition to the use of radioisotopes as detectable elements, which enables in vivo detection by a gamma camera, paramagnetic ions of nonradioactive elements, such as Gd, Fe, Mn or Cr, preferably of Gd, can be used, viz. for in vivo detection by MRI.

For therapeutic purposes the compounds of the invention can advantageously be labelled with therapeutic radionuclides, selected from the group consisting of ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ²¹¹At, ¹¹¹In and ¹⁵³Sm.

Chelation of the above metal isotopes can easily be effected in a manner known per se for related compounds, for example, by bringing the peptide compound of the invention in contact with a compound, often a salt, of the desired isotope in a suitable solvent or diluent, if desired at higher temperature.
The preparation of radioactive-halogen labelled peptide compounds according to the present invention can be carried out by a method as described for related compounds in the above-mentioned WO 00/18440, in order to introduce the desired halogen radionuclide into an aromatic nucleus in position 0 or 1 of the peptide. This labelling can conveniently be performed by introducing a halogen atom or a radioactive halogen atom into an radioactive nucleus, preferably an activated aromatic nucleus such as tyrosyl, present in the above position in the peptide compound, if necessary followed by exchange with the desired halogen radionuclide.

The radiohalogenation reaction is preferably performed by reacting the peptide compound with a solution of an alkali metal radionuclide selected from ¹²³I⁻, ¹³¹I⁻, ²¹¹At⁻, ⁷⁵Br⁻ and ⁷⁶Br⁻ under the influence of a halide-oxidizing agent, such as chloramine T or iodogen. Alternatively, the above substitution reaction can be carried out with nonradioactive halogen, after which halo-exchange with radioactve halogen is performed, e.g. as described in European patent 165630.

The present invention further relates to a pharmaceutical composition, comprising in addition to a pharmaceutically acceptable carrier and, if desired , at least one pharmaceutically acceptable adjuvant, as the active substance a labelled peptide compound as defined above, or a pharmaceutically acceptable salt thereof.
Such pharmaceutical compositions can be used for diagnostic purposes; then the peptide compounds are provided with detectable elements as described above. If the compositions are intended for tumor therapy, advantageously the above-mentioned therapeutic radionuclides can be used for labelling the peptide compounds.

The present invention also relates to a composition for use in a method for detecting and localizing tissues having somatostatin receptors in the body of a warm-blooded living being, This diagnostic method comprises (i) administering to said being a pharmaceutical composition, labelled with a suitable detectable element, as defined above, comprising the active substance in a quantity sufficient for external imaging, and thereupon (ii) subjecting said being to external imaging to determine the targeted sites in the body of said being in relation to the background activity, in order to allow detection and localisation of said tissues in said body.

The present invention further relates to a composition for use in a method for the therapeutic treatment of tumors, having on their surface somatostatin receptors, in the body of a warm-blooded living being, which comprises administering to said being a pharmaceutical composition labelled with a suitable therapeutic radionuclide, as define above, comprising the active substance in a quantity effective for combating or controlling tumors.

It is sometimes hardly possible to put the ready-for use radiolabelled composition at the disposal of the user, in connection with the often poor shelf life of the radiolabelled peptide compound and/or the short half-life of the radionuclide used. In such cases the user him- or herself can carry out the labelling reaction with the radionuclide in the clinical hospital or laboratory. For this purpose the various reaction ingredients are then offered to the user in the form of a so-called "kit". It will be obvious that the manipulations necessary to perform the desired reaction should be as simple as possible to enable the user to prepare from the kit the radioactive-labelled composition by using the facilities that are at his disposal. Therefore the invention also relates to a kit for preparing a radiopharmaceutical composition.

Such a kit according to the present invention may conveniently comprise a peptide compound as defined hereinbefore, viz. derived from a peptide that is or comprises a somatostatin analogue carrying a 3-benzothienylalanine residue in its 3-position, to which substance, if desired, an inert pharmaceutically acceptable carrier and/or formulating agents and/or adjuvants are added, (ii) a solution of a radionuclide compound selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ⁵¹Cr, ²⁴Na, ¹⁵⁷Gd, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh and ¹⁵³Sm, and (iii) instructions for use with a prescription for reacting the ingredients present in the kit. The kit according to the invention preferably comprises a peptide compound derived from a peptide having the general formula I, wherein the symbols have the meanings given hereinbefore.

The invention will now be described in greater detail with reference to the following specific Examples.

### EXAMPLE I

### Synthesis of peptide compounds

The new peptide compounds or peptide conjugates of the invention are synthesized by Fmoc-solid-phase synthesis on 2-chloro-tritylchloride resin (Int. J. Pept. Protein Res. 35, 1990, 161-214). According to this method Fmoc-protected amino-acids are successively coupled, each time followed by cleavage of the protecting Fmoc- group in basic medium. Finally cleavage of the fully protected conjugates from the resin, oxidative cyclization to the cystin-containing cyclic peptide, and introduction of the DOTA-chelator, (e.g. as described by Heppeler et al. in Chem Eur. J. 1999; 5: 1974-1981), leads to the desired peptide compounds comprising the peptides (1) and (2), mentioned hereinbefore, carrying DOTA as the metal-chelating moiety, linked to the free amino group of the peptide. New peptide compound (3) and its conjugate, having the carboxy terminal amino acid group in its carboxylic acid form, as well as the corresponding 3-(1-naphthylalanyl) peptide compound, is prepared as described by Srinivasan et al. in U.S. patent 5,830,431 for corresponding peptide conjugates.

### EXAMPLE II

### Labelling of peptide compounds

The above DOTA carrying peptide compounds are labelled with ¹¹¹In by dissolving each compound in 0.01 M acetic acid, mixing this solution with ¹¹¹InCl₃ -solution (1 mCi / 100µl) in 0.05 M aqueous sodium acetate at higher temperature, and finally neutralizing the solution with HEPES buffer.
Labelling with ⁹⁰Y, obtained from a ⁹⁰Sr - ⁹⁰Y radionuclide generator, is performed as follows. A solution of each of the above DOTA carrying peptide compounds in 0.01 M acetic acid is treated with ⁹⁰Y (1.0 mCi / 50 µl 0.5 M acetate solution). The mixture is left for approx. 1 hr at higher temp. to effect chelation.

### EXAMPLE III

### In vitro binding experiments

In vitro binding affinities are determined using transfected cell lines with somatostatin human receptor subtypes (hsst) 2, 3 and 5, as described by Reubi et al. in Eur. J. Nucl. Med. 27, 2000, 273-282. The affinity profiles (IC₅₀ values), determined for these somatostatin receptor subtypes, are presented in Table A below. In this table the results of a labelled peptide compound according to the invention, viz. ⁸⁹Y-labelled DOTA-(D)Phe-*cyclo*[Cys-3-benzothienylalanyl-(D)Trp-Lys-Thr-Cys]-Thr-ol (Y-DOTA-[BzThi³]-OC; cpd. **10),** are compared with those of Y-DOTA-[1-Nal³]-OC (cpd. 8), referenced to the respective data of somatostatin 28 (cpd. 0).

For purpose of comparison, recently published results (Reubi et al. - see above) of an additional series of IC₅₀ values are also presented in Table A: OC to Y-DOTA-TOC (cpds. 3-6), also referred to the corresponding data of somatostatin S28 (SS-28; cpd. 0).

**Table A**

| | | | | | |
|---|---|---|---|---|---|
| Affinity profiles (IC₅₀) for human sst (hsst) 2, 3 and 5 receptors. | | | | | |
| All values are IC₅₀±SEM in nM. The number of experiments is given in parentheses. | | | | | |
| OC = Octreotide = H-(D)Phe¹-*cyclo*[Cys²-Phe³-(D)Trp⁴-Lys⁵-Thr⁶-Cys⁷]-Thr⁸-ol | | | | | |
| LAN = Lanreotide = H-(D)2-Nal-*cyclo*[Cys-Phe-(D)Trp-Lys-Val-Cys]-Thr-NH₂ | | | | | |
| TOC = H-(D)Phe-*cyclo*[Cys-Tyr-(D)Trp-Lys-Thr-Cys]-Thr-ol | | | | | |

| cpd. no. | compound | hsst 2 | hsst 3 | hsst 4 | hsst 5 |
|---|---|---|---|---|---|
| 0 | SS-28 | 2.7±0.3 | 7.7±0.9 | 1.9±0.9 | 4.0±0.3 |
| 7 | OC | 2.0±0.7 | 187±55 | >1000 | 22±6 |
| 4 | Y-DOTA-OC | 20±2 | 27±8 | >1000 | 57±22 |
| 5 | Y-DOTA-LAN | 23±5 | 290±105 | >1000 | 16±3.4 |
| 6 | Y-DOTA-TOC | 11±1.7 | 389±135 | >10000 | 114±29 |
| 0 | SS-28 | 2.7±0.3 | 3.7±0.3 | 1.9±0.9 | 2.9±0.4 |
| 8 | Y-DOTA-[1-Nal³]-OC | 3.3±0.2 | 26±1.9 | >1000 | 10±1.6 |
| | | | | | |
| **10** | Y-DOTA-[BzThi³]-OC | 3.4 | 13 | | 4.1 |
| **11** | In-DOTA-[BzThi³]-OCate | 1.1 | 7 | 110 | 4 |
| **13** | [BzThi]³-OCate | 0.43 | 27 | 60 | 2.1 |

The above results show that peptide compounds according to the present invention (cpd. **10** and 11) have highly promising affinity profiles with respect to somatostatin receptors. They are binding significantly better to sst 5 than both compounds 5 and 8, and are much better than compounds 4 and 6 on this receptor, even taken into account the different values for the SS-28 (cpd. 0) references. The affinity of cpd. **10** and **11** to sst 3 is also significantly better than for compounds 4 and 8, and even approx. ten times better than for cpd. 5. Surprising is the affinity to the important receptor sst 2. Both compounds have an approx. three times better binding affinity to sst 2 than even cpd. 6. Significantly, both compounds **10** and **11** show an improved binding affinity to sst4. Moreover, the "cold" peptide **13 (BOCate)** has a very high affinity for somatostatin receptors sst 2, 3, 4 and 5. Compound **13** shows a 4 fold higher binding affinity to sst2 than the native peptide and a very high affinity, almost equal to the native peptide to sst5.

### EXAMPLE IV

### Biodistribution studies in vivo

Biodistribution studies are carried out as follows.
Male Lewis rats (180-220 g), bearing the AR42J pancreatic tumor (0.4-3.5 g), are used in the experiments in groups of 4 rats for each time point. The rats are injected subcutaneously (in the abdominal area) under anesthesia with 2-3 MBq of 0.34 nmol (0.5 µg total peptide mass) of the radiolabelled peptide compounds ¹¹¹In-labelled DOTA-(D)Phe-*cyclo*[Cys-3-benzothienylalanyl-(D)Trp-Lys-Thr-Cys]Thr-OH (¹¹¹In-DOTA-[BzThi³]-OCate; cpd. **11**), and, in comparison, ¹¹¹In-DOTA-[1-Nal³]-OCate (cpd. **9**), respectively, both in 0.05 ml saline. After 4 h and 24 h the rats are sacrificed, and the organs and blood are collected and weighed. The radioactivity of these samples is determined using a gamma-counter.

The measured radioactivity, corrected for the weight of the organs and expressed as percentage injected dose per g organ (tissue), is presented in Table B below, together with the standard deviation in parentheses.

**Table B**

| ¹¹¹In-DOTA-[BzThi³]-OCate (compound **11**): | | |
|---|---|---|
| organ/tissue | % i.D./g tissue, after 4 h | % i.D./g tissue, after 24 h |
| blood | 0.025 (± 0.003) | 0.006 (± 0.001) |
| tumor | 4.12 (± 0.62) | 2.05 (± 0.75) |
| kidneys | 1.79 (± 0.15) | 1.83 (± 0.17) |
| adrenals | 5.71 (± 0.53) | 3.34 (± 0.72) |
| pancreas | 10.33 (± 0.34) | 3.30 (± 0.20) |
| stomach | 0.81 (± 0.23) | 0.66 (± 0.36) |
| liver | 0.09 (± 0.01) | 0.07 (± 0.01) |
| | | |

| in comparison: ¹¹¹In-DOTA-[1-Nal³]-OCate (compound **9**): | | |
|---|---|---|
| organ/tissue | % i.D./g tissue, after 4 h | % i.D./g tissue, after 24 h |
| blood | 0.040 (± 0.003) | 0.004 (± 0.001) |
| tumor | 4.01 (± 0.50) | 1.82 (± 0.26) |
| kidneys | 1.51 (± 0.09) | 0.75 (± 0.12) |
| adrenals | 10.76 (± 0.55) | 5.87 (± 1.40) |
| pancreas | 12.31 (± 0.88) | 2.45 (± 0.31) |
| stomach | 1.83 (± 0.62) | 0.92 (± 0.11) |
| liver | 0.09 (± 0.06) | 0.04 (± 0.01) |

From the above results it can be concluded, that for both peptide compounds tested a considerable accumulation of the radioactivity, sufficient for diagnostic purposes and - if labelled with a suitable isotope - for therapeutic purposes, takes place in the tumor tissue, and that the liver metabolic clearance is negligible in comparison with the renal clearance. The renal uptake, marginally (within the error rate) greater for compound **11,** can effectively be suppressed (blocked) by the addition of substances which inhibit the renal uptake: see e.g. Krenning et al. in WO 01/05383. The uptake of radioactive material by the adrenals and the pancreas by using compound **11,** a labelled peptide compound according to the invention, is significantly smaller than the same uptake for compound **9,** a chemically related labelled peptide compound. This gives compound **11** a better tumor to background ratio in the region of the adrenals and the pancreas, allowing effective imaging of these regions. Also the organ accumulation in therapeutic use is less burdening for this peptide compound.

This makes new peptide compounds **10** and **11** of the present invention promising candidates both for imaging purposes and for therapy (of course with a suitable label) of somatostatin- receptor positive tumors.

**Annex to letter dated July 21, 2014 to the European Patent Office concerning European patent application no.** 03722601.6**, publication no.** 1 501 554**, in the name of BioSynthema Inc. et al. - "Peptide compound"**

## Claims

1. A peptide compound having an improved binding affinity to somatostatin receptors, comprising a peptide and a chelating group covalently linked to a free amino group of said peptide, wherein said peptide is or comprises a somatostatin analogue carrying a 3-benzothienylalanine residue in its 3-position,
wherein the peptide has the general formula:
H-(D)A¹-cyclo[Cys²-A³-A⁴-A⁵-A⁶-Cys⁷]-A⁸ (I)
wherein:
A¹ is optionally halogenated Tyr, or optionally halogenated or methylated Phe or Nal,
A⁴ is (D)Trp, optionally N-methylated in its side-chain,
A⁵ is Lys, optionally N-methylated in its side-chain,
A⁶ is Thr, Val, Ser, Phe or Ile, and
A⁸ is Thr, Trp or Nal, wherein the terminal carboxy group may be modified to an alcohol or an , optionally C1-C3 alkylated, amide group,
and **characterized in that** A³ is 3-benzothienylalanyl.

2. A peptide compound as claimed in claim 1, wherein the peptide is a somatostatin analogue of the general formula:
H-(D)Á¹-cyclo[Cys²-A³-(D)Trp⁴-Lys⁵- Á⁶-Cys⁷]- Á⁸ (II)
wherein:
Á¹ is optionally halogenated Tyr, or Phe or Nal,
Á⁶ is Thr or Val, and
Á⁸ is Thr-ol, Thr-OH or Thr-NH2.

3. A peptide compound as claimed in any of the preceding claims, wherein the chelating group is selected from: (i) N2S2-, N3S- and N4-tetradentate ring structure containing groups, (ii) isocyanate, carbonyl, formyl, diazonium, isothiocyanate and alkoxycarbimidoyl containing groups, (iii) groups derived from N-containing di- and polyacetic acids and their derivatives, and from (iv) 2-iminothiolane and 2-iminothiacyclohexane containing groups.

4. A peptide compound as claimed in claim 3, wherein the chelating group is derived from ethylenediamine tetraacetic acid (EDTA), diethylenetriamine pentaacetic acid (DTPA), ethyleneglycol-OO'-bis(2-aminoethyl)-N,N,N',N'-tetraacetic acid (EGTA), N,N'-bis(hydroxylbenzyl)ethylenediamine-N,N'-diacetic acid (HBED), triethylenetetramine hexaacetic acid (TTHA), substituted EDTA or DTPA, 1,4,7,10-tetraazacyclododecane-N,N',N",N"'-tetraacetic acid (DOTA), 1,4,7-triazacyclonane-1,4,7-triacetic acid (NOTA) or 1,4,8,11-tetraazacyclotetradecane-N,N',N",N'''-tetraacetic acid (TETA).

5. A peptide compound as claimed in any of the preceding claims, which compound is labelled with a detectable element selected from gamma- or positron-emitting radionuclides, Auger-electron-emitting isotopes and paramagnetic ions of nonradioactive elements, or with a therapeutic radionuclide.

6. A peptide compound as claimed in claim 5, labelled with a gamma- or positron-emitting radionuclide, selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ⁵¹Cr, ²⁴Na, ¹⁵⁷Gd, ^{52m}Mn, ¹⁶²Dy, ¹²³I, ¹³¹I, ⁷⁵Br and ⁷⁶Br, or with a paramagnetic ion of an element, selected from the group consisting of nonradioactive Gd, Fe, Mn and Cr.

7. A peptide compound as claimed in claim 5, labelled with a therapeutic radionuclide, selected from the group consisting of ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ²¹¹At, ¹¹¹In and ¹⁵³Sm.

8. A pharmaceutical composition comprising in addition to a pharmaceutically acceptable carrier and, if desired , at least one pharmaceutically acceptable adjuvant, as the active substance a labelled peptide compound as claimed in claim 5, or a pharmaceutically acceptable salt thereof.

9. A composition as claimed in claim 8, wherein the peptide compound is labelled with a gamma- or positron-emitting radionuclide, or with a paramagnetic ion of a nonradioactive element, as defined in claim 6.

10. A composition as claimed in claim 8, wherein the peptide compound is labelled with a therapeutic radionuclide as defined in claim 7.

11. A composition as claimed in claim 9, for use in a method for detecting and localizing tissues having somatostatin receptors in the body of a warm-blooded living being, which comprises (i) administering to said being a composition as claimed in claim 9, comprising the active substance in a quantity sufficient for external imaging, and thereupon (ii) subjecting said being to external imaging to determine the targeted sites in the body of said being in relation to the background activity, in order to allow detection and localisation of said tissues in said body.

12. A composition as claimed in claim 10, for use as a medicament.

13. A composition as claimed in claim 10, for use in a method for the therapeutic treatment of tumors having on their surface somatostatin receptors in the body of a warm-blooded living being, which comprises administering to said being a composition as claimed in claim 10, comprising the active substance in a quantity effective for combating or controlling tumors.

14. A kit for preparing a pharmaceutical composition as claimed in claim 8, comprising (i) a peptide compound as claimed in claim 1, to which substance, if desired, an inert pharmaceutically acceptable carrier and/or formulating agents and/or adjuvants are added, (ii) a solution of a radionuclide compound, said radionuclide being selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ⁵¹Cr, ²⁴Na, ¹⁵⁷Gd, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ⁵³Sm, and (iii) instructions for use with a prescription for reacting the ingredients present in the kit.

15. A kit as claimed in claim 14, wherein the peptide compound comprises a peptide as defined in claim 1.

## Patentansprüche

1. Peptidverbindung mit einer verbesserten Bindungsaffinität zu Somatostatinrezeptoren, umfassend ein Peptid und eine chelatbildende Gruppe, die mit einer freien Aminogruppe des Peptids kovalent verbunden ist, wobei das Peptid ein Somatostatin-Analogon ist oder umfasst, welches in seiner 3. Position einen 3-Benzothienylalanin-Rest trägt,
wobei das Peptid die allgemeine Formel aufweist:
H-(D)A¹-cyclo[Cys²-A³-A⁴-A⁵-A⁶-Cys⁷]-A⁸ (I),
worin
A¹ optional halogeniertes Tyr oder optional halogeniertes oder methyliertes Phe oder Na1 ist,
A⁴ (D)Trp ist, welches optional in seiner Seitenkette N-mathyliert ist,
A⁵ Lys ist, welches optional in seiner Seitenkette N-methyliert ist,
A⁶ Thr, Val, Ser, Phe oder Ile ist und
A⁸ Thr, Trp oder Nal ist, wobei die End-Carboxygruppe zu einem Alkohol oder einer optional C1-C3 alkylierten Amidtruppe modifiziert sein kann,
und **dadurch gekennzeichnet,dass**
A³ 3-Benzothienylalanyl ist.

2. Peptidverbindung nach Anspruch 1, wobei das Peptid ein Somatastatin-Analogon der allgemeinen Formel ist:
H-(D)Á¹-cyclo[Cys²-A³-(D)Trp⁴-Lys⁵-Á⁶-Cys⁷]-Á⁸ (II)
worin
Á¹ optional halogeniertes Tyr oder Phe oder Nal ist,
Á⁶ Thr oder Val ist und
Á⁸ Thr-o1, Thr-OH oder Thr-NH2 ist.

3. Peptidverbindung nach einem der vorhergehenden Ansprüche, wobei die chelatbildende Gruppe ausgewählt ist aus:
(i) N2S2-, N3S- und N4-Tetradentat-Ringstruktur enthaltenden Gruppen,
(ii) Isocyanat, Carbonyl, Formyl, Diazonium, Isothiocyanat und Alkoxycarbimidoyl enthaltenden Gruppen,
(iii) Gruppen, die aus N enthaltenden Di- und Polyessigsäuren und deren Derivaten abgeleitet sind, und
(iv) 2-iminothiolan und 2-iminothiacyclohexan enthaltenden Gruppen.

4. Peptidverbindung nach Anspruch 3, wobei die chelatbildende Gruppe aus Ethylendiamin-Tetraessigsäure (EDTA), Diethylentriamin-Pentaessigsäure (DTPA), Ethylenglycol-OO'-bis(2-aminoethyl)-N,N,N',N'-Tetraessigsäure (EGTA), N,N'-bis(hydroxylbenzyl)Ethylendiamin-N,N'-Di-Essigsäure (HBED), Triethylentetramin-Hexaeesigsäure (TTHA), substituierter EDTA oder DTPA, 1,4,7,10-Tetraazacyclododecan-N,N',N",N"'-Tetraessigsäure (DOTA), 1,4,7-Triazacyclonan-1,4,7-Triessigsäure (NOTA) oder 1,4,8,11-Tetraazacylotetradecan-N,N',N",N'''-Tetraessigsäure (TETA) abgeleitet ist.

5. Peptidverbindung nach einem der vorhergehenden Ansprüche, wobei die Verbindung mit einem detektierbaren Element markiert ist, welches aus Gammastrahlen oder Positronen emittierenden Radionukliden, Auger-Elektronen emittierenden Isotopen und paramagnetischen Ionen von nichtradioaktiven Elementen oder mit einem therapeutischen Radionuklid ausgewählt ist.

6. Peptidverbindung nach Anspruch 5, welches mit einem Gammastrahlen oder Positronen emittierenden Radionuklid markiert ist, ausgewählt aus der Gruppe, bestehend aus
^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ⁵¹Cr, ²⁴Na, ¹⁵⁷Gd, ^{52m}Mn, ¹⁶²Dy, ¹²³I, ¹³¹I, ⁷⁵Br und ⁷⁶Br,
oder welches mit einem paramagnetischen Ion eines Elements markiert ist, das aus der Gruppe ausgewählt ist, bestehend aus nichtradioaktivem Gd, Fe, Mn und Cr.

7. Peptidverbindung nach Anspruch 5, markiert mit einem therapeutischen Radionuklid, welches aus der Gruppe ausgewählt ist, bestehend aus:
¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ²¹¹At, ¹¹¹In und ¹⁵³Sm.

8. Pharmazeutische Zusammensetzung, umfassend zusätzlich zu einem pharmazeutisch akzeptablen Träger und, falls erwünscht, zumindest einem pharmazeutisch akzeptablen Adjuvant als aktive Substanz eine markierte Peptidverbindung, wie in Anspruch 5 beansprucht, oder ein pharmazeutisch akzeptables Salz davon.

9. Zusammensetzung nach Anspruch 8, wobei die Peptidverbindung mit einem Gammastrahlen oder Positronenen emittierenden Radionuklid oder mit einem paramagnetischen Ion eines nichtradioaktiven Elements markiert ist, wie in Anspruch 6 festgelegt.

10. Zusammensetzung nach Anspruch 8, wobei die Peptidverbindung mit einem therapeutischen Radionuklid markiert ist, wie in Anspruch 7 festgelegt.

11. Zusammensetzung nach Anspruch 9 zur Verwendung in einem Verfahren zum Detektieren und Lokalisieren von Geweben, welche Somatostatinrezeptoren im Körper eines warmblütigen Lebewesens aufweisen,
wobei das Verfahren umfasst:
(i) Verabreichen dem Lebewesen eine Zusammensetzung, wie in Anspruch 9 beansprucht, umfassend die aktive Substanz in einer für eine externe Bildgebung ausreichenden Menge, und daraufhin
(ii) Unterziehen des Lebewesens einer externen Bildgebung, um die Zielstellen in dem Körper des betreffenden Lebewesens in Bezug auf die Hintergrundaktivität zu bestimmen, um ein Detektieren und Lokalisieren der betreffenden Gewebe in dem Körper zu ermöglichen.

12. Zusammensetzung nach Anspruch 10 für die Verwendung als Medikament.

13. Zusammensetzung nach Anspruch 10 für die Verwendung in einem Verfahren zur therapeutischen Behandlung von Tumoren, die auf ihrer Oberfläche Somatostatinrezeptoren im Körper eines warmblütigen Lebewesens aufweisen, wobei das Verfahren ein Verabreichen einer Zusammensetzung, wie in Anspruch 10 beansprucht, an das Lebewesen umfasst, enthaltend die aktive Substanz in einer Menge, die zum Bekämpfen oder Kontrollieren der Tumoren wirksam ist.

14. Kit zum Herstellen einer pharmazeutischen Zusammensetzung nach Anspruch 8, umfassend:
(i) eine Peptidverbindung, wie in Anspruch 1 beansprucht, der eine Substanz, falls erforderlich, ein inerter pharmazeutisch akzeptabler Träger und/oder Formulierungswirkstoffe und/oder Adjuvanten hinzugesetzt werden,
(ii) eine Lösung einer Radionuklid-Verbindung, wobei das Radionuklid aus der Gruppe ausgewählt ist, bestehend aus:
^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ⁵¹Cr, ²⁴Na, ¹⁵⁷Gd, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ⁵³Sm
und
(iii) Gebrauchsanweisungen mit einer Vorschrift zum Reagierenlassen der im Kit vorhandenen Ingredienzien.

15. Kit nach Anspruch 14, wobei die Peptidverbindung ein Peptid umfasst, wie es in Anspruch 1 festgelegt ist.

## Revendications

1. Composé peptidique présentant une affinité de liaison améliorée aux récepteurs à la somatostatine, comprenant un peptide et un groupe chélateur liés par covalence à un groupe amino libre dudit peptide, dans lequel ledit peptide est ou comprend un analogue de la somatostatine portant un résidu 3-benzothiénylalanine en sa position 3,
dans lequel le peptide possède la formule générale :
H- (D) A¹-cyclo[Cys²-A³-A⁴-A⁵-A⁶-Cys⁷]-A⁸ (I)
dans laquelle :
A¹ représente Tyr éventuellement halogéné, ou Phe ou Nal éventuellement halogéné ou méthylé,
A⁴ représente (D)Trp, éventuellement N-méthylé dans sa chaîne latérale,
A⁵ représente Lys, éventuellement N-méthylé dans sa chaîne latérale,
A⁶ représente Thr, Val, Ser, Phe ou Ile, et
A⁸ représente Thr, Trp ou Nal, dans lequel le groupe carboxy terminal peut être modifié en un alcool ou un groupe amide, éventuellement alkylé par un groupe alkyle en C₁ à C₃,
et **caractérisé en ce que** A³ représente un groupe 3-benzothiénylalanyle.

2. Composé peptidique tel que défini dans la revendication 1, dans lequel le peptide est un analogue de la somatostatine de la formule générale :
H-(D) Á¹-cyclo[Cys²-A³-(D)Trp⁴-Lys⁵-Á⁶-Cys⁷]-Á⁸ (II)
dans laquelle :
Á¹ représente Tyr, ou Phe ou Nal éventuellement halogéné,
Á⁶ représente Thr ou Val, et
Á⁸ représente Thr-ol, Thr-OH ou Thr-NH₂.

3. Composé peptidique tel que défini dans l'une quelconque des revendications précédentes, dans lequel le groupe chélateur est choisi parmi : (i) des groupes contenant une structure de cycle N2S2-, N3S- et N4-tétradenté, (ii) des groupes contenant des groupes isocyanate, carbonyle, formyle, diazonium, isothiocyanate et alcoxycarbimidoyle, (iii) des groupes dérivés de di- et poly-acides acétiques contenant N et de leurs dérivés, et parmi (iv) des groupes contenant des groupes 2-iminothiolane et 2-iminothiacyclohexane.

4. Composé peptidique tel que défini dans la revendication 3, dans lequel le groupe chélateur est dérivé de l'acide éthylène-diamine-tétra-acétique (EDTA), l'acide diéthylène-triamine-penta-acétique (DTPA), l'acide éthylène glycol-00'-bis(2-aminoéthyl)-N,N,N',N'-tétra-acétique (EGTA), l'acide N,N'-bis-(hydroxylbenzyl)éthylènediamine-N,N'-diacétique (HBED), l'acide triéthylène-tétramine-hexa-acétique (TTHA), l'EDTA ou le DTPA substitué, l'acide 1,4,7,10-tétra-azacyclododécane-N,N',N",N"'-tétra-acétique (DOTA), l'acide 1,4,7-triazacyclonane-1,4,7-triacétique (NOTA) ou l'acide 1,4,8,11-tétra-azacyclotétradécane-N,N',N",N'''-tétra-acétique (TETA).

5. Composé peptidique tel que défini dans l'une quelconque des revendications précédentes, lequel composé est marqué avec un élément détectable choisi parmi des radionucléides émetteurs de rayons gamma ou de positons, des isotopes émetteurs d'électrons Auger et des ions paramagnétiques d'éléments non radioactifs, ou avec un radionucléide thérapeutique.

6. Composé peptidique tel que défini dans la revendication 5, marqué avec un radionucléide émetteur de rayons gamma ou de positons, choisi dans le groupe constitué de ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ⁵¹Cr, ²⁴Na, ¹⁵⁷Gd, ^{52m}Mn, ¹⁶²Dy, ¹²³I, ¹³¹I, ⁷⁵Br et ⁷⁶Br, ou avec un ion paramagnétique d'un élément, choisi dans le groupe constitué de Gd, Fe, Mn et Cr non radioactifs.

7. Composé peptidique tel que défini dans la revendication 5, marqué avec un radionucléide thérapeutique, choisi dans le groupe constitué de ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ²¹¹At, ¹¹¹In et ¹⁵³Sm. ¹⁰⁹Pd, ¹⁶⁵Dy, ¹⁷⁷Lu, ¹⁶¹Tb, ^{123m}Rh, ²¹¹At, ¹¹¹In et ¹⁵³Sm.

8. Composition pharmaceutique comprenant, en plus d'un support pharmaceutiquement acceptable, et si souhaité, au moins un adjuvant pharmaceutiquement acceptable, comme substance active un composé peptidique marqué tel que revendiqué dans la revendication 5, ou l'un de ses sels pharmaceutiquement acceptables.

9. Composition telle que définie dans la revendication 8, dans laquelle le composé peptidique est marqué avec un radionucléide émetteur de rayons gamma ou de positons, ou avec un ion paramagnétique d'un élément non radioactif, tel que défini dans la revendication 6.

10. Composition tel que définie dans la revendication 8, dans laquelle le composé peptidique est marqué avec un radionucléide thérapeutique tel que défini dans la revendication 7.

11. Composition telle que définie dans la revendication 9, pour une utilisation dans un procédé de détection et de localisation de tissus comportant des récepteurs à la somatostatine dans le corps d'un être vivant à sang chaud, qui comprend (i) l'administration audit être d'une composition telle que revendiquée dans la revendication 9, comprenant la substance active dans une quantité suffisante pour une imagerie externe, et puis (ii) la soumission dudit être à une imagerie externe pour déterminer les sites ciblés dans le corps dudit être en relation avec l'activité de fond, afin de permettre la détection et la localisation desdits tissus dans ledit corps.

12. Composition telle que définie dans la revendication 10, pour une utilisation en tant que médicament.

13. Composition telle que définie dans la revendication 10, pour une utilisation dans un procédé pour le traitement thérapeutique de tumeurs comportant sur leur surface des récepteurs à la somatostatine dans le corps d'un être vivant à sang chaud, qui comprend l'administration audit être d'une composition telle que revendiquée dans la revendication 10, comprenant la substance active dans une quantité efficace pour combattre ou contrôler des tumeurs.

14. Kit pour la préparation d'une composition pharmaceutique telle que définie dans la revendication 8, comprenant (i) un composé peptidique tel que défini dans la revendication 1, à laquelle substance, si souhaité, un support et/ou des agents de formulation inertes pharmaceutiquement acceptables sont ajoutés, (ii) une solution d'un composé de radionucléide, ledit radionucléide étant choisi dans le groupe constitué de ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ⁵¹Cr, ²⁴Na, ¹⁵⁷Gd, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ⁵³Sm, et (iii) des instructions pour une utilisation avec une prescription pour faire réagir les composants présents dans le kit.

15. Kit tel que défini dans la revendication 14, dans lequel le composé peptidique comprend un peptide tel que défini dans la revendication 1.
